# EUROPEAN PATENT APPLICATION

(11) **EP 2 191 822 A1**
(43) Date of publication of application: **02.06.2010**
(21) Application number: 08169984.5
(22) Date of filing: 26.11.2008
(51) Int. Cl.: A61K 9/20, A61K 9/28, A61K 31/137

(54) **Controlled release pharmaceutical compositions comprising O-desmethyl-venlafaxine**

(71) Applicant: LEK Pharmaceuticals d.d., 1526 Ljubljana (SI)
(72) Inventor: The designation of the inventor has not yet been filed
(74) Representative: Kosak, Alenka

(57) **Abstract**

This invention relates to sustained release pharmaceutical compositions comprising O-desmethyl-venlafaxine, in particular to sustained pharmaceutical compositions comprising O-desmethyl-venlafaxine orotate and / or O-desmethyl-venlafaxine glucuronate.

## Description

### Field of the Invention

This invention relates to sustained release pharmaceutical compositions comprising O-desmethyl-venlafaxine, in particular to sustained pharmaceutical compositions comprising O-desmethyl-venlafaxine orotate and / or O-desmethyl-venlafaxine glucuronate, two new salts of O-desmethyl-venlafaxine.

### Description of the background art

The compound 1-(2-dimethylamino-1-(4-hydroxyphenyl)ethyl)cyclohexanol (Formula 1), generically named O-desmethyl-venlafaxine or desvenlafaxine is the major metabolite of venlafaxine and has been shown to inhibit noradrenaline and serotonine uptake. Its preparation was first disclosed in J. Med. Chem. 1990, 33, pp 2899 in a form of fumarate salt.

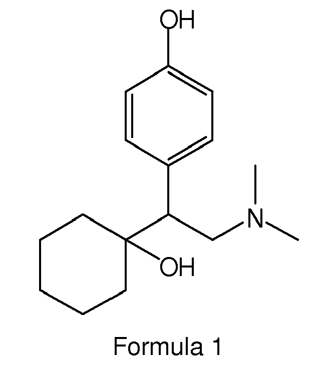

Physical properties of solid pharmaceutical ingredients are essential for preparation of pharmaceutical compositions and its bioavailability. Salts often improve biological characteristics of mother compounds without modifying of primary pharmacological activity, based on mechanism of action. But most of salts are unsuitable due to inappropriate hygroscopicity, stability, solubility, and physically state or simply they cannot be prepared in reasonable yields or cannot be obtained at all.

WO 03/055475 relates to a solid controlled release pharmaceutical formulation comprising a core comprising venlafaxine (notably hydrochloride salt), polyvinylpyrrolidone, a low viscosity hydrophilic polymer and a high viscosity hydrophilic polymer; and a polymeric coating comprising a water high permeable polymer, and a water low permeable polymer.
As desvenlafaxine fumarate has exhibited unsuitable physicochemical and permeability characteristics better forms of desvenlafaxine might be found. A free base is exemplified in WO 00/32555 and stable crystalline forms have been proposed (WO 07/120925). Two new salts such as succinate (WO 02/64543) and formate (WO 03/103603) were also prepared and showed site-specific absorption. Additionally pharmaceutical compositions comprising O-desmethyl-venlafaxine succinate and O-desmethyl-venlafaxine formate are described in WO 02/64543 and formate WO 03/103603, respectively.
WO 07/011619 relates to an oral, highly bioavailable dosage form of O-desmethyl-venlafaxine succinate
Oral administration of O-desmethyl-venlafaxine can result in incidence of nausea, vomiting, diarrhea, and abdominal pain.

The object of the present invention is to provide pharmaceutical compositions suitable and beneficial for sustained release of O-desmethyl-venlafaxine active agent.

### Summary of the invention

The aspects, advantageous features and preferred embodiments of the present invention summarized in the following items, respectively alone or in combination, further contribute to solving the object of the invention:
(1) A sustained release pharmaceutical composition which comprises O-desmethyl-venlafaxine orotate and / or O-desmethyl-venlafaxine glucuronate as active ingredient, and which is defined by a sustained release of the active ingredient.
(2) The pharmaceutical composition according to item (1), wherein the O-desmethyl-venlafaxine salt is O-desmethyl-venlafaxine orotate
(3) The pharmaceutical composition according to item (1), wherein the O-desmethyl-venlafaxine salt is O-desmethyl-venlafaxine glucuronate.
(4) The pharmaceutical composition according to any one of items (1) to (3), comprising a release-rate controlling polymer material.
(5) The pharmaceutical composition according to item (4), wherein said release-rate controlling polymer material is selected from the group consisting of hydrophilic cellulose derivatives (preferably hydroxypropyl methylcellulose, hydroxyethyl cellulose or hydroxypropyl cellulose), hydrophobic cellulose derivatives (preferably ethycellulose), polysaccharides (preferably sodium alginate, propylene glycol alginate, xanthan gum or carrageenans), poly(ethylene) oxides, acrylic polymers and copolymers (preferably carbomers or polymethacrylates), polyvinyl acetate, polyvinylpyrolidone and mixtures thereof.
(6) The pharmaceutical composition according to item (4), wherein said release-rate controlling polymer material is hydrophilic cellulose derivative, preferably selected from the group consisting of hydroxypropyl methylcellulose, hydroxyethyl cellulose and hydroxypropyl cellulose, most preferably hydrophilic cellulose derivative is hydroxypropyl cellulose and / or hydroxypropyl methylcellulose
(7) The pharmaceutical composition according to item (4), wherein said release-rate controlling polymer material is hydrophobic cellulose derivative, preferably ethycellulose.
(8) The pharmaceutical composition according to item (4), wherein said release-rate controlling polymer material is polysaccharide, preferably selected from the group consisting of sodium alginate, propylene glycol alginate, xanthan gum and carrageenan, most preferably polysaccharide is carageenan.
(9) The pharmaceutical composition according to item (4), wherein said release-rate controlling polymer material is poly(ethylene) oxide.
(10) The pharmaceutical composition according to item (4), wherein said release-rate controlling polymer material is acrylic polymer or copolymer, preferably selected from the group consisting of carbomers and polymethacrylates, most preferably acrylic polymer is polymetacrylate.
(11) The pharmaceutical composition according to item (4), wherein said release-rate controlling polymer material is polyvinyl acetate and / or polyvinylpyrrolidone.
(12) The pharmaceutical composition according to any one of items (1) to (11) comprising from about 10 to about 60 % by weight of the rate controlling polymer material, based upon 100 % total weight of said pharmaceutical composition.
(13) The pharmaceutical composition according to any one of items (1 to 12) wherein said pharmaceutical composition is in the form of a tablet.
(14) The pharmaceutical composition according to previous item, wherein the composition is formulated in the tablet core, and the tablet core is further provided with a film coating.
(15) The pharmaceutical composition according to previous item, wherein the film coating comprises a water high permeable polymer and/or a water low permeable polymer.
(16) A pharmaceutical composition comprising O-desmethyl-venlafaxine orotate and / or O-desmethyl-venlafaxine glucuronate as active ingredient in a core, wherein the core is further provided with a film coating comprising at least one water high permeable polymer and at least one water low permeable polymer.
(17) The pharmaceutical composition according to previous two items wherein said at least one water high permeable polymer is selected from the group consisting of hydroxypropyl cellulose, hydroxypropyl methylcellulose, methylcellulose and hydroxyethyl cellulose
(18) The pharmaceutical composition according to any one of the previous three items, wherein said at least one water low permeable polymer is ethylcellulose.
(19) A pharmaceutical composition according to any one of the previous four items, wherein the weight ratio between water high permeable polymer and water low permeable polymer lies in the range of 10 : 1 to 1 : 5.
(20) A pharmaceutical composition according to any one of previous five items, wherein said film coating represents from about 2 to about 10 % of the total weight of said pharmaceutical composition.
(21) The pharmaceutical composition according to any one of the preceding items, wherein the sustained release is characterized by a controlled in vitro release of active ingredient, wherein from about 6 % to about 57 %, preferably from about 25 % to about 45 %, of O-desmethyl-venlafaxine is released after 2 hours, when tested using USP Apparatus 1, placing the pharmaceutical composition either in 900 ml water with 0.9% NaCl, or in 900 ml 0.01 M HCl for 60 minutes and subsequently in 900 ml water with 0.9% NaCl, at 37ºC with paddle speed of 150 rpm,
   wherein the %-values define the ratio of released active ingredient in relation to the amount originally contained in the pharmaceutical composition.
(22) The pharmaceutical composition according to any one of the preceding items, characterized by controlled in vitro release of active ingredient, wherein from about 21 % to about 72 %, preferably from about 35 % to about 65 %, of O-desmethyl-venlafaxine is released after 4 hours.
(23) The pharmaceutical composition according to any one of the preceding items, characterized by a controlled in vitro release of active ingredient, wherein from about 45 % to about 96 %, preferably from about 55 % to about 85 %, of O-desmethyl-venlafaxine is released after 8 hours.
(24) The pharmaceutical composition according to any one of the preceding items, characterized by a controlled in vitro release of active ingredient, wherein more than 61 %, preferably more than 70 %, of O-desmethyl-venlafaxine is released after 12 hours.
(25) The pharmaceutical composition according to any one of the preceding items, characterized by a controlled in vitro release of active ingredient, wherein more than 73 %, preferably more than 80 % of O-desmethyl-venlafaxine is released after 16 hours.
(26) A pharmaceutical composition according to any one of preceding items, wherein the sustained release is characterized by a controlled in vitro release of active ingredient, wherein from about 6 % to about 57 %, preferably from about 25 % to about 45 %, of O-desmethyl-venlafaxine is released after 2 hours, from about 21 % to about 72 %, preferably from about 35 % to about 65 %, of O-desmethyl-venlafaxine is released after 4 hours, from about 45 % to about 96 %, preferably from about 55 % to about 85 %, of O-desmethyl-venlafaxine is released after 8 hours, more than 61 %, preferably more than 70 %, of O-desmethyl-venlafaxine is released after 12 hours and more than 73 %, preferably more than 80 % of O-desmethyl-venlafaxine is released after 16 hours, when tested using USP Apparatus 1, placing the pharmaceutical composition either in 900 ml water with 0.9% NaCl, or in 900 ml 0.01 M HCl for 60 minutes and subsequently in 900 ml water with 0.9% NaCl, at 37ºC with paddle speed of 150 rpm,
   wherein the %-values define the ratio of released active ingredient in relation to the amount originally contained in the pharmaceutical composition.
(27) A sustained release pharmaceutical composition comprising O-desmethyl-venlafaxine orotate and / or O-desmethyl-venlafaxine glucuronate as active ingredient, characterized by a controlled in vitro release of active ingredient, wherein from about 6 % to about 57 % of O-desmethyl-venlafaxine is released after 2 hours, from about 21 % to about 72 % of O-desmethyl-venlafaxine is released after 4 hours, from about 45 % to about 96 % of O-desmethyl-venlafaxine is released after 8 hours, more than 61 % of O-desmethyl-venlafaxine is released after 12 hours and more than 73 %, of O-desmethyl-venlafaxine is released after 16 hours, when tested using USP Apparatus 1, placing the pharmaceutical composition either in 900 ml water with 0.9% NaCl, or in 900 ml 0.01 M HCl for 60 minutes and subsequently in 900 ml water with 0.9% NaCl, at 37ºC with paddle speed of 150 rpm,
   wherein the %-values define the ratio of released active ingredient in relation to the amount originally contained in the pharmaceutical composition.
(28) The sustained release pharmaceutical according to the previous item, more specifically characterized by controlled in vitro release of active ingredient, wherein from about 25 % to about 45 % of O-desmethyl-venlafaxine is released after 2 hours, from about 35 % to about 65 % of O-desmethyl-venlafaxine is released after 4 hours, from about 55 % to about 85 % of O-desmethyl-venlafaxine is released after 8 hours, more than 70 % of O-desmethyl-venlafaxine is released after 12 hours and more than 80 % of O-desmethyl-venlafaxine is released after 16 hours, when tested using USP Apparatus 1, placing the pharmaceutical composition either in 900 ml water with 0.9% NaCl, or in 900 ml 0.01 M HCl for 60 minutes and subsequently in 900 ml water with 0.9% NaCl, at 37ºC with paddle speed of 150 rpm.
(29) A set or package including a predeterminded number of pharmaceutical compositions respectively comprising O-desmethyl-venlafaxine orotate and / or O-desmethyl-venlafaxine glucuronate as active ingredient and respectively having the same composition, preferably in the form of tablets, wherein each sample of the set or package provides the release profile indicated in any one of items (21) to (28).
(30) O-desmethyl-venlafaxine orotate or O-desmethyl-venlafaxine glucuronate for use as a medicament.
(31) O-desmethyl-venlafaxine orotate or O-desmethyl-venlafaxine glucuronate formulated into a sustained release pharmaceutical composition for use as a medicament.

### Description of further Advantages and Preferred Embodiments of the Invention:

In the following, the present invention will be described in more detail by preferred embodiments and examples while referring to the attached drawings, noting, however, that these embodiments, examples and drawings are presented for illustrative purposes only and shall not limit the invention in any way.

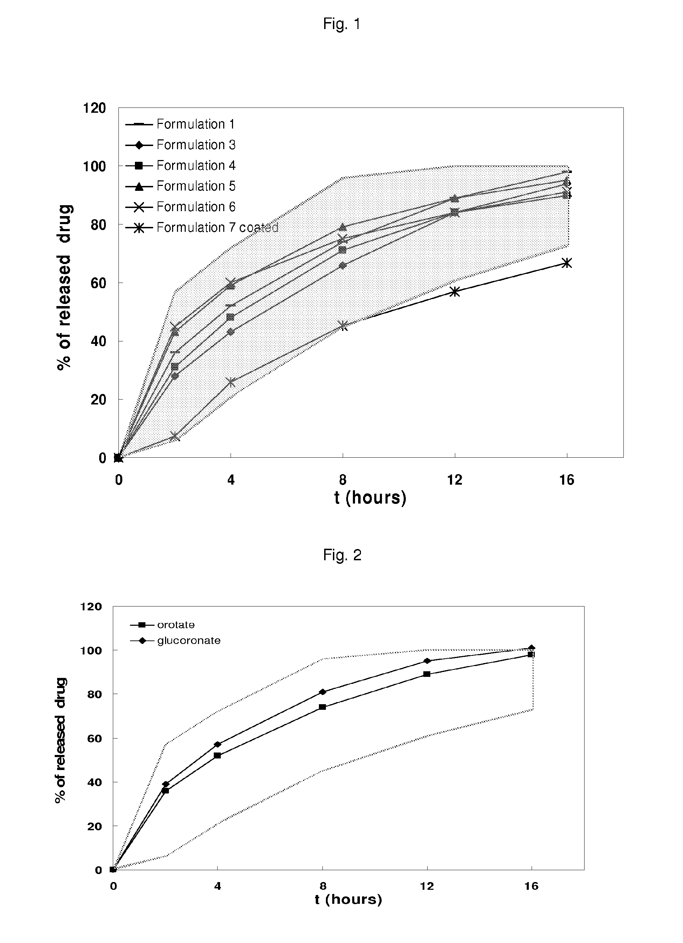

Fig. 1 shows dissolution profiles of sustained release compositions comprising O-desmethyl-venlafaxine orotate prepared according to Examples 1, 3, 4, 5, 6 and 7. In grey the possible release profile range that gives satisfactory biological effect but does not exceed 225 ng / ml of O-desmethyl-venlafaxine in the plasma, is marked. Fig. 2 shows dissolution profiles of sustained release compositions comprising O-desmethyl-venlafaxine glucurontate in comparison with the same formulation comprising O-desmethyl-venlafaxine orotate, respectively prepared in accordance with Example 1.
In grey the possible release profile range that gives satisfactory biological effect but does not exceed 225 ng / ml of O-desmethyl-venlafaxine in the plasma, is marked.

The term "about" generally means within 10%, preferably 5% and more preferably within 1 % of a given value or range. Alternatively, the term "about" means within an acceptable standard error of the mean, when considered by one of the ordinary skill in the art.

Oral administration of O-desmethyl-venlafaxine can result in incidence of nausea, vomiting, diarrhea, and abdominal pain that can be avoided by different physico-chemical properties of specifically selected O-desmethyl-venlafaxine salts and can be further controlled by designing special final dosage forms including sustained release formulations of such selected O-desmethyl-venlafaxine salts. Different from previously described salts of succinate and formate, the O-desmethyl-venlafaxine orotate and O-desmethyl-venlafaxine glucuronate salts share a common structural feature of having a relatively bulk salt moiety. Further both the orotate salt partner and the glucuronate salt partner are physiologically highly acceptable.

Selection of either O-desmethyl-venlafaxine orotate or O-desmethyl-venlafaxine glucuronate, or using both salts in combination, enables to provide useful sustained release of the O-desmethyl-venlafaxine active agent from corresponding sustained release pharmaceutical compositions. In particular, selection of one or both of the specified salts beneficially makes it possible to adjust and repeatedly give a suitable release profile and thus to control the balance of effectivity and adverse side-effects. When desired and demanded, oral dosage forms can be designed that same or similar release profiles can be obtained in a repeatable manner.

The present invention makes use of newly discovered salts of O-desmethyl-venlafaxine, selected from O-desmethyl-venlafaxine orotate and O-desmethyl-venlafaxine glucuronate. In one specific embodiment, the O-desmethyl-venlafaxine salt is O-desmethyl-venlafaxine orotate. In another specific embodiment, the O-desmethyl-venlafaxine salt is O-desmethyl-venlafaxine glucuronate. These particular salts O-desmethyl-venlafaxine orotate and glucuronate have useful properties for drug formulation. They are both very soluble in water. O-desmethyl-venlafaxine orotate solubility in water is 30 mg/ml. O-desmethyl-venlafaxine glucuronate is 10 times more soluble in water than orotic salt. The pH (1% water solution) of orotate is 3.3 and of glucoronate 6.14.
Based on these findings and beneficial physico-chemical properties of specifically selected O-desmethyl-venlafaxine salts the present invention provides in a first aspect a sustained release pharmaceutical composition comprising the O-desmethyl-venlafaxine salt disclosed herein as active ingredient. In particular sustained release pharmaceutical composition according to the present invention comprises O-desmethyl-venlafaxine orotate and / or O-desmethyl-venlafaxine glucuronate and a release-rate controlling polymer material. Owing to the particular combination of the specifically selected O-desmethyl-venlafaxine salts and the release-rate controlling polymer material, the pharmaceutical compositions according to present invention show small variations in drug release with regards to dissolution media and with regards to active ingredient incorporated. In order to test the influence of physiochemical differences of O-desmethyl-venlafaxine orotate salt compared to O-desmethyl-venlafaxine succinate on release from the matrix formulations, identical formulations using with O-desmethyl-venlafaxine succinate and O-desmethyl-venlafaxine orotate were prepared. Dissolution of O-desmethyl-venlafaxine orotate salt was much faster comparing to O-desmethyl-venlafaxine succinate from the tablet having the same composition. This is surprising since the solubility in water of both salts is similar (30,3 mg/ml for orotate and 29,2 mg/ml for succinate). Pharmaceutical compositions according to present invention diminish these differences, showing that compositions can be prepared that are less susceptible to physiochemical differences of incorporated active ingredient in terms of drug release. Therefore, selection of O-desmethyl-venlafaxine orotate and / or O-desmethyl-venlafaxine glucuronate enables a large freedom to choose among further possible additives and excipients and still establish desirable and useful characteristics of the pharmaceutical composition.

As noted above, oral administration of O-desmethyl-venlafaxine can result in incidence of nausea, vomiting, diarrhea, and abdominal pain. It is believed that adverse effects are related to the too high peak blood plasma level and / or tₘₐₓ of the O-desmethyl-venlafaxine.
Above-mentioned side-effects can be reduced thanks to different physico-chemical properties of O-desmethyl-venlafaxine salts and due to designing special final dosage forms including sustained release oral formulations.

The present invention provides sustained release oral pharmaceutical compositions comprising O-desmethyl-venlafaxine orotate and / or O-desmethyl-venlafaxine glucuronate, two newly discovered salts of O-desmethyl-venlafaxine. Pharmaceutical compositions according to present invention show desirable dissolution properties and according to in vitro/in vivo correlation show favorable peak blood plasma levels of O-desmethyl-venlafaxine and at the same time have reduced adverse effects that are related to the high peak blood plasma level. That is, the salts O-desmethyl-venlafaxine orotate and / or O-desmethyl-venlafaxine glucuronate are useful in allowing good balance between drug activity and control of adverse effects. In addition pharmaceutical compositions according to present invention show small variations in drug release with regards to dissolution media and with regards to active ingredient incorporated, good stability and are also characterized by simple manufacturing procedure.

Pharmaceutical compositions according to present invention may suitably comprise from about 10 % to about 70 % of O-desmethyl-venlafaxine orotate and / or O-desmethyl-venlafaxine glucuronate, preferably from about 20 % to about 50 %, based upon 100 % total weight of said pharmaceutical composition. Coating if present is included in total weight of pharmaceutical composition.

In one aspect the present invention relates to sustained release pharmaceutical compositions comprising O-desmethyl-venlafaxine orotate and / or O-desmethyl-venlafaxine glucuronate and a release-rate controlling polymer material.

Due to very good solubility of O-desmethyl-venlafaxine salts in water, from the outset it may be difficult to prepare sustained release formulations that could efficiently enable desired release. The most straightforward solution for too fast release of very water soluble active substances from sustained release matrix tablets is increasing the amount of rate controlling polymer material content and increasing the mass of the tablets. In the view of the situation that relatively larger tablets are less preferred in terms of patient compliance, the amount of excipients should be controlled. According to the present invention, appropriate release-rate controlling polymer material, or combination of rate controlling polymer materials that act synergistically, and/or provision of the active ingredient in core combined with an appropriate film coating were found as critical factors - each alone and preferably in combination further contributing to display desirable drug-release profiles.

The term "sustained release" used herein means a retarded or prolonged release of the O-desmethyl-venlafaxine from the pharmaceutical composition, for example longer than when only the O-desmethyl-venlafaxine active ingredient compound as such is tested in a given test solution.

The term "release-rate controlling polymer" used herein means a polymer which accomplishes a retarded or prolonged release of the O-desmethyl-venlafaxine active ingredient from the pharmaceutical composition, relative to a direct or immediate release defined by unaffected dissolution under a given condition. The polymer may form a matrix controlling the release of the active ingredient from the pharmaceutical composition.

Various useful rate controlling polymer materials according to present invention include but are not limited to the following:
in one embodiment the polymer is one of hydrophilic cellulose derivatives, preferably hydroxypropyl methylcellulose, hydroxyethyl cellulose or hydroxypropyl cellulose, most preferably hydroxypropyl cellulose and / or hydroxypropyl methylcellulose;
in another embodiment the polymer is one of hydrophobic cellulose derivatives, preferably ethycellulose;
in a further embodiment the polymer is one of polysaccharides, preferably sodium alginate, propylene glycol alginate, xanthan gum or carrageenans, most preferably carageenan;
in still another embodiment the polymer is one of poly(ethylene) oxides;
according to a still further embodiment the polymer is one of acrylic polymers and copolymers, preferably carbomers or polymethacrylates;
another embodiment of the polymer is polyvinyl acetate;
still a further embodiment the polymer is polyvinylpyrrolidone.
Included are also mixtures of two or more of the aforementioned polymers, i.e. combinations from the same or from different types of polymers.

In a certain embodiment according to present invention, pharmaceutical composition comprises from about 10 to about 60 % by weight of the rate controlling polymer material, based upon 100 % total weight of said pharmaceutical composition.

The pharmaceutical composition of the present invention may further comprise additional pharmaceutically acceptable excipients. Pharmaceutically acceptable excipients include but are not limited to such as binders, fillers, glidants, anticaking agents, lubricants, lipid matrix forming agents, etc.

Various useful binders include but are not limited to hydroxypropyl cellulose, hydroxypropyl methylcellulose, polyvinylpyrrolidone, powdered acacia, gelatin, guar gum, carbomer (e.g. Carbopol), methylcellulose, polymethacrylates, and starch. Preferred binder is polyvinylpyrrolidone.

Various useful fillers include but are not limited to calcium phosphate dehydrate, starches, lactose, mannitol, cellulose derivatives and the like. Different grades of lactose include but are not limited to lactose monohydrate and lactose anhydrous. Different grades of starches included but are not limited to maize starch, potato starch, rice starch, wheat starch, pregelatinized starch, fully pregelatinized starch. Different cellulose compounds that can be used include microcrystalline cellulose and powdered cellulose. Preferred fillers are calcium phosphate dehydrate and / or microcrystalline cellulose.

Various useful glidants include but are not limited to starches, colloidal silicon dioxide and talc. Preferred glidants are colloidal silicon dioxide or talc.

Various useful anticaking agents include but are not limited to colloidal silicon dioxide and talc. Preferred anticaking agent is talc.

Various suitable lubricants include but are not limited to stearic acid, talc and magnesium stearate. Preferred lubricant is magnesium stearate.

Lipid matrix forming agent is preferably glyceryl behenate.

When rate controlling polymer material is poly(ethylene oxide), solution of NaCO₃ is used as granulation solution that inhibits the rapid hydration of poly(ethylene oxide) during granulation process.

A pharmaceutical composition according to the present invention is preferably in solid form, including tablets, capsules, caplets, lozenges and sachets. Capsule formulations may cover both soft and hard capsules. A pharmaceutical composition according to the present invention is preferably in the form of tablet. Said tablet is preferably film coated. The film coating may comprise a water high permeable polymer and/or a water low permeable polymer, which will be described in further detail also below in connection with another aspect of the invention that particularly deals with the coating film design as an alternative to control release of the active ingredient.

In a further aspect of the present invention, the pharmaceutical composition comprises O-desmethyl-venlafaxine orotate and / or O-desmethyl-venlafaxine glucuronate as active ingredient in a core, wherein the core is further provided with a film coating comprising at least one water high permeable polymer and at least one water low permeable polymer. This aspect alone, or in combination with the above described aspects and features, contributes to a beneficial influence on a desired sustained dissolution profile.

The term "water high permeable polymer" used herein shall mean a polymer which is soluble in water, suitably determined by a weight-% percentage of dissolution in water of 3.3% or more, more suitably 5% or more, even more suitably 10% or more, particularly suitably 50% or more and especially suitably 70% or more, for example hydroxypropyl cellulose, hydroxypropyl methylcellulose, methylcellulose and hydroxylethyl cellulose, or which can achieve water permeability by swelling or salt formation, for example methacrylate-aminoester copolymer or methylcellulose, or which contains groups permeable to water or binding water molecules in a relatively high proportion of e.g. a molar ratio of water permeable to water non-permeable groups being 1:30 or more, for example high permeable poly(ethylacrylate methylmethacrylate) trimethylammoniumethylmethacrylate chloride; or the like.

The term "water low permeable polymer" used herein shall mean a polymer which is essentially insoluble in water, including ones which are insoluble at physiological pH, for example ethylcellulose, and ones which are insoluble in acidic pH, for example cellulose acetate phthalate, methacrylic acid copolymers, polyvinyl acetate phthalate, cellulose acetate trimellitate, hydroxypropyl methylcellulose phthalate, or which contains groups permeable to water or binding water molecules in a relatively low proportion of e.g. a molar ratio of water permeable to water non-permeable groups being 1:30 or less, for example low permeable poly (ethylacrylate, methylmethacrylate) trimethylammoniumethylmethacrylate chloride; or the like.

Water high permeable polymer may preferably be selected from the group consisting of methylcellulose, hydroxypropyl cellulose, hydroxypropyl methylcellulose, hydroxyethyl cellulose, methacrylate aminoester copolymer, high permeable poly (ethylacrylate, methylmethacrylate) trimethylammoniumethylmethacrylate chloride, preferably hydroxypropyl cellulose, hydroxypropyl methylcellulose, methylcellulose and high permeable poly (ethylacrylate, methylmethacrylate) trimethylammoniumethylmethacrylate chloride, more preferably from hydroxypropyl cellulose, hydroxypropyl methylcellulose, methylcellulose or hydroxyethyl cellulose, most preferably hydroxypropyl cellulose, without however being restricted to these examples.

Water low permeable polymer may preferably be selected from the groups consisting of ethylcellulose, cellulose acetate phthalate, methacrylic acid copolymers, polyvinyl acetate phthalate, cellulose acetate trimellitate, hydroxypropyl methylcellulose phthalate and low permeable poly (ethylacrylate, methylmethacrylate) trimethylammoniumethylmethacrylate chloride, preferably ethylcellulose, hydroxypropyl methylcellulose phthalate, polyvinyl acetate phthalate and low permeable poly (ethylacrylate, methylmethacrylate) trimethylammoniumethylmethacrylate chloride, most preferably from ethylcellulose; without however being restricted to these examples.

Preferred combinations of water high permeable and water low permeable polymers may be selected in particular from, but not limited to, combination of hydroxypropyl methylcellulose and hydroxypropyl methylcellulose phthalate, combination of hydroxypropyl cellulose and hydroxypropyl methylcellulose phthalate, combination of hydroxypropyl methylcellulose and ethylcellulose, combination of hydroxypropyl cellulose and ethylcellulose, combination of hydroxypropyl methylcellulose and polyvinyl acetate phthalate, combination of hydroxypropyl cellulose and polyvinyl acetate phthalate, combination of high permeable poly (ethylacrylate, methylmethacrylate) trimethylammoniumethylmethacrylate chloride and low permeable poly (ethylacrylate, methylmethacrylate) trimethylammoniumethylmethacrylate chloride. A particularly preferred combination is one of hydroxylpropyl methylcellulose and hydroxypropyl methylcellulose phthalate, one of hydroxylpropyl cellulose and ethylcellulose, one of hydroxypropyl methylcellulose and ethylcellulose, and one of high permeable poly (ethylacrylate, methylmethacrylate) trimethylammoniumethylmethacrylate chloride and low permeable poly (ethylacrylate, methylmethacrylate) trimethylammoniumethylmethacrylate chloride.
In the embodiments of combinations, the ratio between the water high permeable and water low permeable polymers is preferably from 10:1 to 1:5, more preferably from 6:1 to 1:4, particularly preferably from 3:1 to 1:3 and specifically said ratio is about 1 : 1.

In another embodiment said film coating represents from about 2 to about 10 %, preferably from about 4 to about 6 %, of the total weight of pharmaceutical composition.

In another aspect the present invention relates to a sustained release pharmaceutical composition comprising O-desmethyl-venlafaxine orotate and / or O-desmethyl-venlafaxine glucuronate, wherein the sustained release is characterized by a controlled in vitro release of active ingredient as defined by any one of the following release rates, alone or in combination:
from about 6 % to about 57 %, preferably from about 25 % to about 45 %, of O-desmethyl-venlafaxine is released after 2 hours;
from about 21 % to about 72 %, preferably from about 35 % to about 65 %, of O-desmethyl-venlafaxine is released after 4 hours;
from about 45 % to about 96 %, preferably from about 55 % to about 85 %, of O-desmethyl-venlafaxine is released after 8 hours;
more than 61 %, preferably more than 70 %, of O-desmethyl-venlafaxine is released after 12 hours;
more than 73 %, preferably more than 80 % of O-desmethyl-venlafaxine is released after 16 hours;
when respectively tested using USP Apparatus 1, placing the pharmaceutical composition either in 900 ml water with 0.9% NaCl, or in 900 ml 0.01 M HCl for 60 minutes and subsequently in 900 ml water with 0.9% NaCl, at 37ºC with paddle speed of 150 rpm,
wherein the %-values define the ratio of released active ingredient in relation to the amount originally contained in the pharmaceutical composition.
More preferably all in vitro release rate conditions of active ingredient are met in that from about 6 % to about 57 %, preferably from about 25 % to about 45 %, of O-desmethyl-venlafaxine is released after 2 hours, from about 21 % to about 72 %, preferably from about 35 % to about 65 %, of O-desmethyl-venlafaxine is released after 4 hours, from about 45 % to about 96 %, preferably from about 55 % to about 85 %, of O-desmethyl-venlafaxine is released after 8 hours, more than 61 %, preferably more than 70 %, of O-desmethyl-venlafaxine is released after 12 hours and more than 73 %, preferably more than 80 % of O-desmethyl-venlafaxine is released after 16 hours, when tested using USP Apparatus 1, placing the pharmaceutical composition either in 900 ml water with 0.9% NaCl, or in 900 ml 0.01 M HCl for 60 minutes and subsequently in 900 ml water with 0.9% NaCl, at 37ºC with paddle speed of 150 rpm, wherein the %-values define the ratio of released active ingredient in relation to the amount originally contained in the pharmaceutical composition..

In the above-referenced dissolution test with the USP Apparatus 1, using a first solution of 900 ml 0.01 M HCl for 60 minutes and subsequently a solution of 900 ml water with 0.9% NaCl is suitably chosen when the pharmaceutical composition is provided with an acid soluble coating film, whereas a single test solution of 900 ml water with 0.9% NaCl is suitable in other cases. In either case, a paddle speed of 150 rpm is suitably used in the dissolution test, and temperature condition is at 37ºC. Said acid soluble coating film for example comprises dialkylaminoalkylacrylic copolymer (such as for example Eudragit E PO or Eudragit E 100)

A developed *in vitro*-*in vivo* correlation model that links blood plasma concentration of O-desmethyl-venlafaxine with the *in vitro* dissolution release rate of O-desmethyl-venlafaxine orotate or glucuronate salts from various sustained release compositions showed that above mentioned controlled in vitro release of active ingredient is critical in order not to exceed peak blood plasma concentration of 225 ng / ml of O-desmethyl-venlafaxine but still to achieve satisfactory biological effect. Higher peak blood plasma concentrations of O-desmethyl-venlafaxine are namely connected with increased frequency of side effects, such as nausea and emesis. The above mentioned controlled in vitro release profiles from pharmaceutical compositions according to the present invention are therefore very useful in terms of both efficacy and control of adverse effects.

According to another aspect, the present invention provides a set or package including a predeterminded number of pharmaceutical compositions respectively comprising O-desmethyl-venlafaxine orotate and / or O-desmethyl-venlafaxine glucuronate as active ingredient and respectively having the same formulation composition including the same excipients besides the active ingredient, preferably in the form of tablets, wherein each sample of the set or package provides the above mentioned controlled in vitro release profile. The term "a set or package including a predeterminded number of pharmaceutical compositions" used herein means samples collected from different production samples, batches or lots, or samples collected from a medical package unit. Variation within the set may be calculated from 10 different samples such as 10 tablets. According to the present invention, it is made possible to satisfy the variation characteristics even between different production batches/lots, and between samples contained in a given medical package. This characteristic is indicative of reproducibly obtaining desired and beneficial in vitro release profiles for samples throughout a set or package of pharmaceutical compositions, notably tablets.

The pharmaceutical compositions containing the new salts O-desmethyl-venlafaxine orotate and / or O-desmethyl-venlafaxine glucuronate as disclosed herein are useful as medicalment: For example they can be used for the prophylaxis and/or the treatment of depression (such as major depressive disorder, bipolar disorder, and dysthymia), anxiety, panic disorder, generalized anxiety disorder, post traumatic stress disorder, premenstrual dysphoric disorder, fibromyalgia, agorophobia, attention deficit disorder (with and without hyperactivity), obsessive compulsive disorder (including trichotillomania), social anxiety disorder, autism, schizophrenia, obesity, anorexia nervosa, bulimia nervosa, Gilles de la Tourette Syndrome, vasomotor flushing, cocaine and alcohol addiction, sexual dysfunction (such as premature ejaculation), borderline personality disorder, chronic fatigue syndrome, urinary incontinence, pain (such as migraine, chronic back pain, phantom limb pain, central pain, neuopathic pain such as diabetic neuropathy, and postherpetic neuropathy), Shy Drager syndrome, Raynaud's syndrome, Parkinson's disease, hypothalamic amenorrhea in depressed and non-depressed human females, and epilepsy. Treatment may include administering to a patient in need thereof, an effective amount of O-desmethyl-venlafaxine orotate or O-desmethyl-venlafaxine glucuronate, or mixtures thereof.

### Examples

Selected formulations are given with short description of preparation process. The tablets were compressed at a weight from 350 to 400 mg using round biconvex punches.

### Example 1 (Formulation 1): Hydrophilic cellulose matrix tablets

| ***Composition*** | ***function*** | ***mg*** | ***%*** |
|---|---|---|---|
| O-desmethyl-venlafaxine orotate | active | 159.00 | 45.43 |
| Hydroxypropyl cellulose | delaying matrix polymer | 120.00 | 34.28 |
| Polyvinylpyrrolidone (PVP) | binder | 8.00 | 2.29 |
| Microcrystalline cellulose | filler | 61.00 | 17.43 |
| Magnesium stearate | lubricant | 2.00 | 0.57 |
| | | *350.00* | *100.00* |

- O-desmethyl-venlafaxine orotate is mixed with hydroxypropyl cellulose and part of microcrystalline cellulose. The mixture is granulated with PVP water solution in a high shear mixer or fluid bed.
- The granulate is dried in oven or a fluid bed dryer.
- Dry mixture is sieved through appropriate sieve.
- Microcrystalline cellulose and magnesium stearate are added to granulate and mixed together and tablets are compressed.

Other hydrophilic cellulose polymers can be used instead of hydroxypropyl cellulose (such as for example hydroxypropyl methylcellulose, hydroxyethyl cellulose).

### Example 2 (Formulation 2): Water-insoluble (hydrophobic) cellulose matrix tablets

| ***Composition*** | ***function*** | ***mg*** | ***%*** |
|---|---|---|---|
| O-desmethyl-venlafaxine orotate | active | 159.00 | 45.43 |
| Ethylcellulose | delaying matrix polymer | 130.00 | 37.15 |
| Polyvinylpyrrolidone (PVP) | binder | 5.25 | 1.50 |
| Dicalcium phosphate dehydrate | filler | 53.75 | 15.35 |
| Magnesium stearate | lubricant | 2.00 | 0.57 |
| | | *350.00* | *100.00* |

• O-desmethyl-venlafaxine orotate is mixed with ethylcellulose (ethylcelluloses of different viscosity can be used), microcrystalline cellulose and PVP. The mixture is granulated with water in a high shear mixer.
• The granulate is dried in oven or a fluid bed dryer.
• Dry mixture is sieved through appropriate sieve.
• Magnesium stearate is added to granulate and mixed together and tablets are compressed.

### Example 3 (Formulation 3): Matrix tablets of polysaccharides

| ***Composition*** | ***function*** | ***mg*** | ***%*** |
|---|---|---|---|
| O-desmethyl-venlafaxine orotate | active | 159.00 | 45.43 |
| Microcrystalline cellulose | filler | 14.00 | 4.00 |
| Hydroxypropyl cellulose | release matrix former | 70.00 | 20.00 |
| Carrageenan Gelcarin GP-379 | delaying matrix polymer | 52.50 | 15.00 |
| Carrageenan Viscarin GP-209 | delaying matrix polymer | 52.50 | 15.00 |
| Magnesium stearate | lubricant | 2.00 | 0.57 |
| | | *350.00* | *100.00* |

- O-desmethyl-venlafaxine orotate is mixed with hydroxypropyl cellulose and microcrystalline cellulose. The mixture is granulated with water in a high shear mixer or fluid bed.
- The granulate is dried in oven or a fluid bed dryer.
- Dry mixture is sieved through appropriate sieve.
- Carrageenans and magnesium stearate are added to granulate and mixed together and tablets are compressed.

Other polysaccharides can be used instead of carrageenans (such as for example sodium alginates, propylene glycol alginate, xanthan).

### Example 4 (Formulation 4):Poly(ethylene) oxide matrix tablets

| ***Composition*** | ***function*** | ***mg*** | ***%*** |
|---|---|---|---|
| O-desmethyl-venlafaxine orotate | active | 159.00 | 45.43 |
| Poly (ethylene) oxide | delaying matrix polymer | 130.80 | 37.37 |
| Microcrystalline cellulose | filler | 37.40 | 10.69 |
| Dicalcium phosphate dihydrate | filler | 18.80 | 5.37 |
| Sodium carbonate | granulation enhancer | 2.00 | 0.57 |
| Magnesium stearate | lubricant | 2.00 | 0.57 |
| | | *350.00* | *100.00* |

- O-desmethyl-venlafaxine orotate is mixed with poly(ethylene) oxide (poly(ethylene) oxide of different viscosity can be used), microcrystalline cellulose and dicalcium phosphate dihydrate.
- The mixture is granulated with sodium carbonate water solution in a high shear mixer.
- The granulate is dried in oven or a fluid bed dryer.
- Dry mixture is sieved through appropriate sieve.
- If necessary the granulate was milled before sieving.
- Magnesium stearate is added to granulate and the tablets are compressed.

### Example 5 (Formulation 5): Tablets with matrix of acrylic polymers

| ***Composition*** | ***function*** | ***mg*** | ***%*** |
|---|---|---|---|
| O-desmethyl-venlafaxine orotate | active | 159.00 | 39.75 |
| Eudragit^{®} NE 30D and/or Eudragit^{®} L 100-55 | delaying matrix polymer | 150.00 | 37.50 |
| Microcrystalline cellulose | filler | 45.40 | 11.35 |
| Dicalcium phosphate dihydrate | filler | 39.30 | 9.83 |
| Talc | glidant | 4.00 | 1.00 |
| Magnesium stearate | lubricant | 2.30 | 0.57 |
| | | *400.00* | *100.00* |

- O-desmethyl-venlafaxine orotate is mixed with dicalcium phosphate dihydrate and microcrystalline cellulose mixture is granulated with Eudragit dispersion in fluid bed.
- The granulate is dried in a fluid bed dryer.
- Dry mixture is sieved through appropriate sieve.
- If necessary the granulate was milled before sieving.
- In some cases additional Eudragit was added to prepared granulate. Magnesium stearate and talc are also added to granulate and the mixture is compressed.

Other polymethacrylates can be used instead of said Eudragits. Also acrylic acid polymers - carbomers (such as for example Carbopol 971 P, Carbopol 71 G) can be used.

### Example 6 (Formulation 6: Polyvinyl acetate/polyvinylpyrolidone matrix tablets

| ***Composition*** | ***function*** | ***mg*** | ***%*** |
|---|---|---|---|
| O-desmethyl-venlafaxine orotate | Active | 159.00 | 39.75 |
| Polyvinyl acetate/ polyvinyl-pyrolidone mixture (Kollidon SR^{®}) | delaying matrix polymer | 184.75 | 46.19 |
| Glyceryl behenate | Lipid matrix forming agent | 53.25 | 13.31 |
| Colloidal silicon dioxide | glidant | 1.00 | 0.25 |
| Magnesium stearate | lubricant | 2.00 | 0.50 |
| | | *400.00* | *100.00* |

- O-desmethyl-venlafaxine orotate is mixed with polyvinyl acetate/polyvinylpyrolidone mixture, glyceryl behenate, aerosil and magnesium stearate: The mixture is compressed.
- To improve the flowability of powder mixture in some cases the tablets were prepared by dry granulation.

### Example 7 (Formulation 7): Coated matrix tablets

| ***Composition*** | ***function*** | ***mg*** | ***%*** |
|---|---|---|---|
| ***Tablet core using the composition of Example 6*** | | 350.00 | 95.63 |
| ***Coat*** | | | |
| Ethylcellulose N7 | film forming polymer | 5.35 | 1.46 |
| Hydroxypropyl cellulose Klucel EF | film forming polymer | 5.35 | 1.46 |
| Triethyl citrate | plasticizer | 0.96 | 0.26 |
| Titanium dioxide | colorant | 3.27 | 0.90 |
| Talc | anticaking agent | 1.07 | 0.29 |
| | | *366.00* | *100.00* |

Matrix tablets were also film coated with polymeric coating to reach optimal dissolution profile. Different weight percentages of coating per total weight of the formulation were applied. The coating is presented in the formulation in the range of 2 - 10% of the total weight of the formulation. The coating comprises a combination of two different polymers; a water high permeable (hydroxypropyl cellulose, hydroxypropyl methylcellulose, methylcellulose and hydroxyethyl cellulose) and a water low permeable polymers (ethylcellulose). The combination of these two polymers may be selected for particular coating with specific weight ratio between the polymers (ratio from 10:1 to 1:5 of water high permeable and low permeable polymers).

### Example 8

Tablets were produced in the same manner and with the same pharmaceutical composition as in Example 1 except that O-desmethyl-venlafaxine orotate was replaced by O-desmethyl-venlafaxine glucuronate.

### Dissolution tests:

Dissolution properties were evaluated by *in vivo* relevant dissolution test. USP apparatus 1 was used for testing. 900 ml of water with 0.9% NaCl is placed in the dissolution vessel, mixed with a paddle at 150 rpm and kept at 37 ± 0.5°C. Samples were taken from the dissolution vessel at regular time intervals and the concentrations of O-desmethyl-venlafaxine were analyzed by HPLC.

Pharmaceutical compositions might be optionally placed for 60 minutes in 900 ml 0.01 M HCl before being placed in 900 ml water with 0.9% NaCl.

Dissolution profiles for sustained release pharmaceutical compositions prepared according to Examples 1 to 7 (shown in Figure 1) show efficient control of the release of O-desmethyl-venlafaxine orotate. It becomes apparent that the dissolution profiles of all illustrated examples can be controlled to lie in the grey region, and therefore, according to our developed *in vitro* - *in vivo* correlation model, it is expected on the one hand to give satisfactory biological effect but on the other hand does not exceed 225 ng / ml of O-desmethyl-venlafaxine in the plasma. Suitable sustained release tablets using different applicable release-rate controlling polymers are already achieved by uncoated tablets (see formulations of Examples 1 to 6). An additional provision of a film coating onto a core containing a release-rate controlling polymer shows some further retardation of release (see formulation of Example 7). A film coating onto a core therefore provides for an additional control factor affecting drug release. Moreover, the additional effect demonstrated by the formulation of Example 7 compared to the one of Example 6 makes credible that the combination of at least one water high permeable polymer and at least one water low permeable polymer added to a film coating provides a useful release-rate control of its own when combined with a O-desmethyl-venlafaxine salt selected from O-desmethyl-venlafaxine orotate and O-desmethyl-venlafaxine glucuronate being added to the core.

Sustained release compositions comprising O-desmethyl-venlafaxine glucurontate instead of O-desmethyl-venlafaxine orotate but otherwise using the same formulation show similar sustained dissolution profiles, as demonstrated by a comparison using as the exemplified formulation the one of Example 1 illustrated in Fig. 2.

## Claims

1. A sustained release pharmaceutical composition which comprises O-desmethyl-venlafaxine orotate and / or O-desmethyl-venlafaxine glucuronate as active ingredient, and which is defined by a sustained release of the active ingredient.

2. The pharmaceutical composition according to claim 1, comprising a release-rate controlling polymer material.

3. The pharmaceutical composition according to claim 2, wherein said release-rate controlling polymer material is selected from the group consisting of:
hydrophilic cellulose derivatives, preferably hydroxypropyl methylcellulose, hydroxyethyl cellulose or hydroxypropyl cellulose;
hydrophobic cellulose derivatives, preferably ethycellulose;
polysaccharides, preferably sodium alginate, propylene glycol alginate, xanthan gum or carrageenans;
poly(ethylene) oxides;
acrylic polymers or copolymers, preferably carbomers or polymethacrylates;
polyvinyl acetate;
polyvinylpyrolidone;
and mixtures thereof.

4. The pharmaceutical composition according to claim 2 or 3, comprising from about 10 to about 60 % by weight of the release-rate controlling polymer material, based upon 100 % total weight of said pharmaceutical composition.

5. The pharmaceutical composition according to any one of the preceding claims, wherein said pharmaceutical composition is in the form of a tablet, optionally as a tablet core further provided with a film coating.

6. The pharmaceutical composition according to claim 5, wherein the film coating comprises a water high permeable polymer and/or a water low permeable polymer.

7. A pharmaceutical composition comprising O-desmethyl-venlafaxine orotate and / or O-desmethyl-venlafaxine glucuronate as active ingredient in a core, wherein the core is further provided with a film coating comprising at least one water high permeable polymer and at least one water low permeable polymer.

8. The pharmaceutical composition according to claim 7, wherein said at least one water high permeable polymer is selected from the group consisting of hydroxypropyl cellulose, hydroxypropyl methylcellulose, methylcellulose and hydroxyethyl cellulose, and/or wherein said at least one water low permeable polymer is ethylcellulose.

9. A pharmaceutical composition according to any one of claims 6 to 8, wherein the weight ratio between water high permeable polymer and water low permeable polymer lies in the range of 10 : 1 to 1 : 5.

10. A pharmaceutical composition according to any one of claims 5 to 9, wherein said film coating represents from about 2 to about 10 % of the total weight of said pharmaceutical composition.

11. The pharmaceutical composition according to any one of the preceding claims, wherein the sustained release is **characterized by** a controlled in vitro release of active ingredient as defined by any one of the following release rates, alone or in combination:
from about 6 % to about 57 %, preferably from about 25 % to about 45 %, of O-desmethyl-venlafaxine is released after 2 hours;
from about 21 % to about 72 %, preferably from about 35 % to about 65 %, of O-desmethyl-venlafaxine is released after 4 hours;
from about 45 % to about 96 %, preferably from about 55 % to about 85 %, of O-desmethyl-venlafaxine is released after 8 hours;
more than 61 %, preferably more than 70 %, of O-desmethyl-venlafaxine is released after 12 hours;
more than 73 %, preferably more than 80 % of O-desmethyl-venlafaxine is released after 16 hours;
when respectively tested using USP Apparatus 1, placing the pharmaceutical composition either in 900 ml water with 0.9% NaCl, or in 900 ml 0.01 M HCl for 60 minutes and subsequently in 900 ml water with 0.9% NaCl, at 37ºC with paddle speed of 150 rpm,
wherein the %-values define the ratio of released active ingredient in relation to the amount originally contained in the pharmaceutical composition.

12. A sustained release pharmaceutical composition comprising O-desmethyl-venlafaxine orotate and / or O-desmethyl-venlafaxine glucuronate as active ingredient, **characterized by** a controlled in vitro release of active ingredient, wherein from about 6 % to about 57 % of O-desmethyl-venlafaxine is released after 2 hours, from about 21 % to about 72 % of O-desmethyl-venlafaxine is released after 4 hours, from about 45 % to about 96 % of O-desmethyl-venlafaxine is released after 8 hours, more than 61 % of O-desmethyl-venlafaxine is released after 12 hours and more than 73 %, of O-desmethyl-venlafaxine is released after 16 hours, when tested using USP Apparatus 1, placing the pharmaceutical composition either in 900 ml water with 0.9% NaCl, or in 900 ml 0.01 M HCl for 60 minutes and subsequently in 900 ml water with 0.9% NaCl, at 37ºC with paddle speed of 150 rpm,
wherein the %-values define the ratio of released active ingredient in relation to the amount originally contained in the pharmaceutical composition.

13. A set or package including a predeterminded number of pharmaceutical compositions respectively comprising O-desmethyl-venlafaxine orotate and / or O-desmethyl-venlafaxine glucuronate as active ingredient and respectively having the same composition, wherein each sample of the set or package provides the release profile indicated in claim 12.

14. O-desmethyl-venlafaxine orotate or O-desmethyl-venlafaxine glucuronate formulated into a sustained release pharmaceutical composition for use as a medicament.

15. O-desmethyl-venlafaxine orotate or O-desmethyl-venlafaxine glucuronate formulated into a sustained release pharmaceutical composition for use in the prophylaxis and/or the treatment of depression, anxiety, panic disorder, neuropathic pain, post traumatic stress disorder, premenstrual disorder, postmenopausal syndrome, fibromyalgia, agorophobia, attention deficit disorder, obsessive compulsive disorder, autism, schizophrenia, obesity, anorexia nervosa, bulimia nervosa, Gilles de la Tourette Syndrome, vasomotor flushing, cocaine and alcohol addiction, sexual dysfunction, borderline personality disorder, chronic fatigue syndrome, urinary incontinence, pain, Shy Drager syndrome, Raynaud's syndrome, Parkinson's disease, hypothalamic amenorrhea in depressed and non-depressed human, and epilepsy.
